# EUROPEAN PATENT SPECIFICATION

(11) **EP 1 562 611 B1**
(45) Date of publication and mention of the grant of the patent: **02.08.2006**
(21) Application number: 03775585.7
(22) Date of filing: 19.11.2003
(51) Int. Cl.: A61K 31/695, A61K 31/341, A61K 31/381, A61K 31/4025, C07F 7/08, C07D 207/32, C07D 307/46, C07D 333/22

(54) **HETEROCYCLIC SILICON COMPOUNDS AND THEIR USE IN THE TREATMENT OF DISEASES OR CONDITIONS ASSOCIATED WITH GNRH (GONADOTROPIN-RELEASING HORMONE)**
HETEROZYKLISCHE SILIZIUMVERBINDUNGEN UND DEREN VERWENDUNG ZUR BEHANDLUNG VON KRANKHEITEN ODER ZUSTÄNDEN, DIE MIT GONADOTROPIN-FREISETZENDEN HORMON ASSOZIIERT SIND
COMPOSES DE SILICIUM HETEROCYCLIQUES ET UTILISATION DE CEUX-CI DANS LE TRAITEMENT DE MALADIES OU D'ETATS ASSOCIES A GNRH (HORMONE DE LIBERATION DE LA GONADOTROPINE)

(30) Priority: 20.11.2002 GB 0227131; 07.05.2003 GB 0310472; 23.07.2003 GB 0317283
(43) Date of publication of application: 17.08.2005
(73) Proprietor: Paradigm Therapeutics Ltd., 418 Cambridge Science Park Milton Road Cambridge CB4 0PA (GB)
(72) Inventor: MONTANA, John Gary, Paradigm Therapeutics Ltd., Cambridge CB4 0PA (GB); FLEMING, Ian, University Chemical Laboratory, Cambridge CB2 1EW (GB); TACKE, Reinhold, 97249 Eisingen (DE); DAISS, Jurgen, 97074 Würzburg (DE)
(74) Representative: Perry, Robert Edward
(86) International application number: PCT/GB2003/005015
(87) International publication number: WO 2004/045625

(56) References cited:
- WO-A-00/20358
- WO-A-03/068769
- WO-A-03/106446
- ANDERES KENNA L ET AL: "Biological characterization of a novel, orally active small molecule gonadotropin-releasing hormone (GnRH) antagonist using castrated and intact rats." THE JOURNAL OF PHARMACOLOGY AND EXPERIMENTAL THERAPEUTICS, vol. 305, no. 2, May 2003 (2003-05), pages 688-695, XP009026574 ISSN: 0022-3565
- LUTHIN DAVID R ET AL: "Characterization of mono- and diaminopyrimidine derivatives as novel, nonpeptide gonadotropin releasing hormone (GnRH) receptor antagonists." BIOORGANIC & MEDICINAL CHEMISTRY LETTERS, vol. 12, no. 24, 16 December 2002 (2002-12-16), pages 3635-3639, XP002271752 ISSN: 0960-894X
- LUTHIN DAVID R ET AL: "The discovery of novel small molecule non-peptide gonadotropin releasing hormone (GnRH) receptor antagonists." BIOORGANIC & MEDICINAL CHEMISTRY LETTERS, vol. 12, no. 23, 2 December 2002 (2002-12-02), pages 3467-3470, XP002271753 ISSN: 0960-894X
- IATSIMIRSKAIA EUGENIA A ET AL: "Effect of testosterone suppression on the pharmacokinetics of a potent gnRH receptor antagonist." PHARMACEUTICAL RESEARCH, vol. 19, no. 2, February 2002 (2002-02), pages 202-208, XP009026578 ISSN: 0724-8741
- TACKE R ET AL: "Sila-substitution--a useful strategy for drug design?" ENDEAVOUR, vol. 10, no. 4, 1986, pages 191-197, XP008002622 ISSN: 0160-9327

## Description

This invention relates to silicon compounds and their use in therapy.

### Background to the Invention

Gonadotropin-releasing hormone (GnRH) plays a key role in the biology of reproduction. GnRH is also known as luteinising hormone-releasing hormone (LH-RH).

The GnRH decapeptide (pyro-Glu-His-Trp-Ser-Tyr-Gly-Leu-Art-Pro-Gly-NH₂ or p-EHWSYGLRPG-NH₂) is formed in neurons of the medical basal hypothalamus from a larger precursor via enzymatic processing. The peptide is released in a pulsatile manner into the pituitary portal circulation system, where it interacts with high-affinity receptors (7-transmembrane G-protein coupled receptors) in the anterior pituitary gland located at the base of the brain. Here, GnRH triggers the release of luteinising hormone (LH) and follicle-stimulating hormone (FSH), both of which are gonadotropic hormones. LH stimulates the production of testosterone and estradiol in the testes and ovaries respectively, whilst FSH stimulates follicle growth in women and sperm formation in men. When correctly functioning, the pulsatile release and concentration levels of GnRH are critical for maintaining gonadal steroidogenesis and the normal functions of reproduction related to growth and sexual development.

The pituitary response to GnRH varies greatly throughout life. GnRH and the gonadotropins first appear in the foetus at about ten weeks of gestation. Sensitivity to GnRH reduces until the onset of puberty but there is, however, a brief rise during the first three months after birth. Prior to puberty, the FSH response to GnRH is greater than that of LH. Once puberty begins, sensitivity to GnRH increases, and pulsatile LH secretion ensues. Later, in puberty and throughout the reproductive years, pulsatile release of GnRH occurs throughout the day, with the responsiveness to LH greater than that to FSH. Pulsatile GnRH release results in pulsatile LH and FSH release and, in turn, pulsatible testosterone and estradiol release from the gonads. Post-menopause, the concentration of FSH an LH rise, and the post-menopausal levels of FSH are higher than those of LH.

Chronic administration of GnRH agonists and antagonists results in decreased circulating levels of both LH and FSH.

GnRH agonists are compounds that mimic endogenous GnRH to stimulate receptors on the pituitary gland, resulting in release of LH and FSH. After a transient rise in gonadal hormone production (a "flare" response), the chronic administration of GnRH agonists results in down-regulation of the GnRH receptors. This down-regulation and desensitisation results in a reduction in the circulating levels of LH and FSH. In spite of the symptom-exacerbating hormonal flare experienced, GnRH agonists have been the preferred treatment for sex-steroid-dependent pathophysiologies. GnRH agonists have been used to reduce testosterone production, thereby reducing prostate volume in benign prostatic hyperplasia (BPH) and slowing tumour growth in prostate cancer. Such compounds have also been used in the treatment of cancer, for example breast and ovarian cancers.

In recent years, GnRH antagonists have become available for clinical evaluation, and have been shown to have an immediate effect on the pituitary but without the observed flare associated with agonists. Use of GnRH antagonists has been reported for the treatment of ovarian, breast and prostate cancers. Other uses of antagonists include the treatment of endometriosis (including endometriosis with pain), uterine myoma, ovarian and mammary cystic diseases (including polycystic ovarian disease), prostatic hypertrophy, amenorrhea (e.g. secondary amenorrhea), precocious puberty and in the symptomatic relief of premenstrual syndrome (PMS). Antagonists may also be useful to regulate the secretion of gonadotropins in male mammals to arrest spermatogenesis (e.g. as male contraceptives), and for the treatment of male sex offenders. GnRH antagonists and agonists have been shown to have utility in treatments where a reversible suppression of the pituitary-gonadal axis is desired.

Conventionally, androgen deprivation has been the most effective systematic therapy for the treatment of metastatic carcinoma of the prostate. The prostate gland requires androgens for normal growth, maintenance, and function. Prostate cancer and benign prostate hyperplasia, however, are common in men and develop in an environment of continuous exposure to androgen. Utilising a GnRH antagonist to interrupt the pituitary-gonadal axis reduces androgen production and results in tumour growth modulation.

GnRH antagonists may have a direct effect on tumour growth by blocking receptors on the tumour cells. For those cancer types that respond both to sex hormones and to GnRH directly, GnRH antagonists should be effective in stowing tumour growth by two mechanisms. Since GnRH receptors are present on many prostate and breast cancer cells, it has recently been proposed that GnRH antagonists may also be effective in treating non-hormone-dependent tumours. Recent literature examples indicate that GnRH receptors are present on a number of cancer cell lines, in particular, prostate, ovarian and breast cancers (see for example Montagnani *et al, Arch. Ital, Urol. Androl.* **1997,** 69(4), 257-263; Jungwirth *et al, Prostate* **1997,** 32(3), 164-172; Srkalovic *et al, Int. J. Oncol.* **1998,** 12(3), 489-498; and Koftler *et al, Int. J. Cancer* **1997,** *71 (4),* 595-599.

To date, GnRH antagonists have primarily been peptide analogues of GnRH (see, for example, WO93/03058). Such antagonists of peptide hormones have some potency but are often associated with problems because the peptides are degraded by physiological enzymes and often poorly distributed within the organism being treated. They thus have a limited effectiveness as drugs.

WO00/20358 discloses non-peptide analogues of GnRH.

Sila-substitution (C/Si-exchange) of drugs is a relatively recent approach for searching for organosilicon compounds which have beneficial biological properties. The approach involves the replacement of specific carbon atoms in compounds by silicon, and monitoring how the biological properties of the compounds have changed. A review of this approach is provided in Tacke and Zilch, Endeavour, New Series, 10, 191-197 (1986).

### Summary of the Invention

The present invention concerns small-molecule non-peptidic GnRH antagonists that may exploit both of the above-described mechanisms of action. Such non-peptidic agents may have advantageous physical, chemical and biological properties compared to peptides, and may be useful as medicaments for diseases such as those mediated via the pituitary-gonadal axis and by directly targeting the receptor on tumour cells.

According to a first aspect of the invention, a compound has the formula (I) 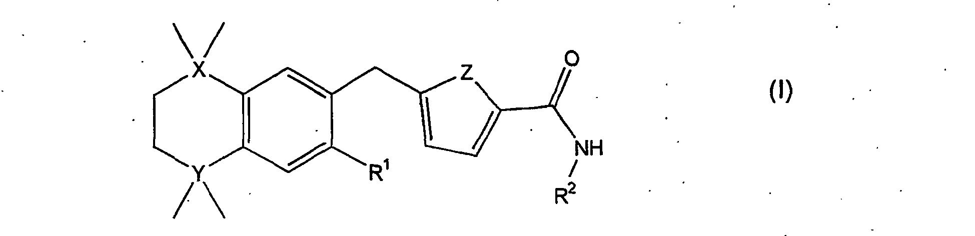 wherein
one of X and Y is silicon, and the other is carbon or silicon;
Z is oxygen, sulphur or -N(R)-, wherein R is hydrogen or alkyl;
R¹ is hydrogen, halogen, alkyl, alkenyl, alkynyl, alkoxy or cycloalkyl; and
R² is alkyl, alkenyl, alkynyl, cycloalkyl, heterocycloalkyl, aryl, heteroaryl, -alkyl-cycloalkyl, -alkyl-heterocycloalkyl, -alkyl-aryl or -alkyl-heteroaryl;
or a pharmaceutically acceptable salt thereof.

Another aspect of the invention is a pharmaceutical composition comprising a compound of formula (I) and a pharmaceutically acceptable diluent or carrier.

Compounds of the invention may act as GnRH antagonists. Accordingly, another aspect of the invention is the use of a compound of formula (I) for the manufacture of a medicament for the treatment or prevention of a disease or condition associated with GnRH. The compounds may have utility in the treatment or prevention of a fertility disorder, Alzheimer's disease, HIV infection, AIDS, fibrosis, endometriosis, uterine fibroids, uterine leiomyoma or cancer (e.g. leukemia).

The compounds may have a better biodistribution and tolerance to degradation by physiological enzymes, and thus may be pharmaceutically advantageous over peptide compounds.

### Description of the Preferred Embodiments

Certain compounds and combinations of substituents are preferred; in particular see the subclaims.

With regard to formula (I), X and Y are preferably each silicon. Z is preferably oxygen. R¹ is preferably hydrogen or methyl. R² is preferably aryl, -CH₂-cycloalkyl, -CH₂-aryl, -CH₂-heterocycloalkyl or -CH₂-heteroaryl. More preferably, R² is phenyl optionally substituted, for example with one, two or three alkoxy groups.

A preferred compound of the invention is 5-[(3,5,5,8,8-pentamethyl-5,8-disila-5,6,7,8-tetrahydro-2-naphthyl)methyl]-*N*-(2,4,6-trimethoxyphenyl)furan-2-carboxamide.

The term "alkyl" as used herein refers to an optionally substituted straight or branched chain alkyl moiety having from one to six carbon atoms. The term includes, for example, methyl, ethyl, propyl, isopropyl, butyl, tert-butyl, pentyl and hexyl. The group may be optionally substituted with one or more substituents, the substituents being the same or different in each occurrence and selected from hydroxy, halogen and the like. "C₁₋₆ alkyl" has the same meaning.

The term "alkenyl" as used herein refers to an optionally substituted straight or branched chain alkyl moiety having from two to six carbon atoms and having in addition at least one double bond, of either E or Z stereochemistry where applicable. This term includes, for example, vinyl, 1-propenyl, 1- and 2-butenyl, 2- methyl-2-propenyl etc. The group may be optionally substituted with one or more substituents, the substituents being the same or different in each occurrence and selected from hydroxy and the like. "C₂₋₆ alkenyl" has the same meaning.

The term "alkynyl" as used herein refers to an optionally substituted straight or branched chain alkyl moiety having two to six carbon atoms and having in addition at least one triple bond. The group may be optionally substituted with one or more substituents, the substituents being the same or different in each occurrence and selected from hydroxy and the like. "C₂₋₆ alkynyl" has the same meaning.

The term "alkoxy" as used herein refers to an optionally substituted straight chain or branched chain alkoxy group containing between one and six carbon atoms, such as methoxy, ethoxy, propoxy, isopropoxy, butoxy, tert-butoxy and the like. The group may be optionally substituted with one or more substituents, the substituents being the same or different in each occurrence and selected from halogen and the like. "C₁₋₆ alkoxy" has the same meaning.

The term "aryl" as used herein refers to an optionally substituted aromatic ring system comprising six to ten ring atoms or an optionally substituted polycyclic ring systems having two or more rings, at least one of which is aromatic. This term includes for example, phenyl and naphthyl. The group may be optionally substituted with one or more substituents, the substituents being the same or different in each occurrence and selected from halogen, alkyl, alkenyl, alkynyl, hydroxyl, alkoxy, silyloxy, amino, nitro, sulphydryl, alkylthio, amido, phosphoryl, phosphonate, phosphino, carbonyl, carboxyl, carboxamido, alkylsilyl, thioalkyl, alkylsulphonyl, arylsulfonyl, selenoalkyl, ketone, ester, heteroalkyl, cyano, guanidino, amidino, acetal, ketal; amine oxide, aryl, heteroaryl, arylalkyl, heteroarylalkyl, carbamate, hydroxamic acid, imido; sulphonamido, thioamido, thiocarbamate, urea and thiourea.

The term "cycloalkyl" as used herein refers to an optionally substituted saturated alicyclic moiety having from three to six carbon atoms. The term includes, for example, cyclopropyl, cyclobutyl, cyclopentyl, cyclohexyl and the like. The group may be optionally substituted with one or more substituents, the substituents being the same or different in each occurrence and selected from hydroxy, alkoxy, amino, amido and the like.

The term "heterocycloalkyl" as used herein refers to an optionally substituted saturated heterocyclic moiety having from four to seven carbon atoms and one or more heteroatoms selected from the group N, O, S, P and Si, and includes, for example, azetidinyl, pyrrolidinyl, tetrahydrofuranyl, piperidinyl and the like. The group may be optionally substituted by any substituent described herein with one or more substituents, the substituents being the same or different in each occurrence and selected from hydroxy, alkoxy, amino, amido and the like.

The term "heteroaryl" as used herein refers to an optionally substituted aromatic ring system of five to ten atoms at least one of which is selected from O, N and S, and includes, for example, furanyl, thiophenyl, pyridyl, indolyl, quinolyl and the like. The group may be optionally substituted by any substituent described herein with one or more substituents, the substituents being the same or different in each occurrence and selected from halogen, alkyl, alkenyl, alkynyl, hydroxyl, alkoxy, silyloxy, amino, nitro, sulphydryl, alkylthio, amido, phosphoryl, phosphonate, phosphino, carbonyl, carboxyl, carboxamido, alkylsilyl, thioalkyl, alkylsulphonyl, arylsulfonyl, selenoalkyl, ketone, ester, heteroalkyl, cyano, guanidino, amidino, acetal, ketal, amine oxide, aryl, heteroaryl, arylalkyl, heteroarylalkyl, carbamate, hydroxamic acid, imido, sulphonamido, thioamido, thiocarbamate, urea and thiourea.

The term "halogen" as used herein refers to F, Cl, Br or I.

Compounds of the invention may be chiral. They may be in the form of a single enantiomer or diastereomer, or a racemate.

The compounds of the invention may be prepared in racemic form, or prepared in individual enantiomeric form by specific synthesis or resolution as will be appreciated in the art. The compounds may, for example, be resolved into their enantiomers by standard techniques, such as the formation of diastereomeric pairs by salt formation with an optically active acid followed by fractional crystallisation and regeneration of the free base. Alternatively, the enantiomers of the novel compounds may be separated by HPLC using a chiral column.

A compound of the invention may be in a protected amino, protected hydroxy or protected carboxy form. The terms "protected amino", "protected hydroxy" and "protected carboxy" as used herein refer to amino, hydroxy and carboxy groups which are protected in a manner familiar to those skilled in the art. For example, an amino group can be protected by a benzyloxycarbonyl, tert-butoxycarbonyl, acetyl or like group, or in the form of a phthalimido or like group. A carboxyl group can be protected in the form of a readily cleavable ester such as the methyl, ethyl, benzyl or tert-butyl ester. A hydroxy group can be protected by an alkyl or like group.

Compounds of the invention may be in the form of pharmaceutically acceptable salts, for example, addition salts of inorganic or organic acids. Such inorganic acid addition salts include, for example, salts of hydrobromic acid, hydrochloric acid, nitric acid, phosphoric acid and sulphuric acid. Organic acid addition salts include, for example, salts of acetic acid, benzenesulphonic acid, benzoic acid, camphorsulphonic acid, citric acid, 2-(4-chlorophenoxy)-2-methylpropionic acid, 1,2-ethanedisulphonic acid, ethanesulphonic acid, ethylenediaminetetraacetic acid (EDTA), fumaric acid, glucoheptonic acid, gluconic acid, glutamic acid, 4-hexylresorcinol, hippuric acid, 2-(4-hydroxybenzoyl)benzoic acid, 1-hydroxy-2-naphthoic acid, 3-hydroxy-2-naphthoic acid, 2-hydroxyethanesulphonic acid, lactobionic acid, n-dodecyl sulphuric acid, maleic acid, malic acid, mandelic acid, methanesulphonic acid, methyl sulphuric acid, mucic acid, 2-naphthalenesulphonic acid, pamoic acid, pantothenic acid, phosphanilic acid ((4-aminophenyl)phosphonic acid), picric acid, salicylic acid, stearic acid, succinic acid, tannic acid, tartaric acid, terephthalic acid, p-toluenesulphonic acid, 10-undecenoic acid and the like.

Salts may also be formed with inorganic bases. Such inorganic base salts include, for example, salts of aluminium, bismuth, calcium, lithium, magnesium, potassium, sodium, zinc and the like. Organic base salts include, for example, salts of N, N'-dibenzylethylenediamine, choline (as a counterion), diethanolamine, ethanolamine, ethylenediamine, N,N'-bis(dehydroabietyl)-ethylenediamine, N-methylglucamine; procaine, tris(hydroxymethyl)aminomethane ("TRIS") and the like.

A compound of the invention may be prepared by any suitable method known in the art and/or by the following processes:

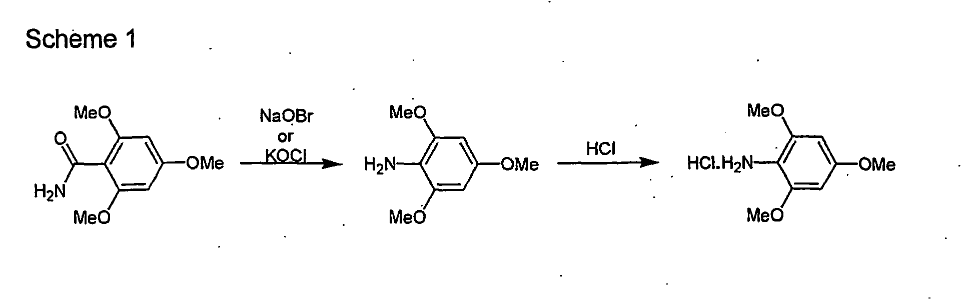

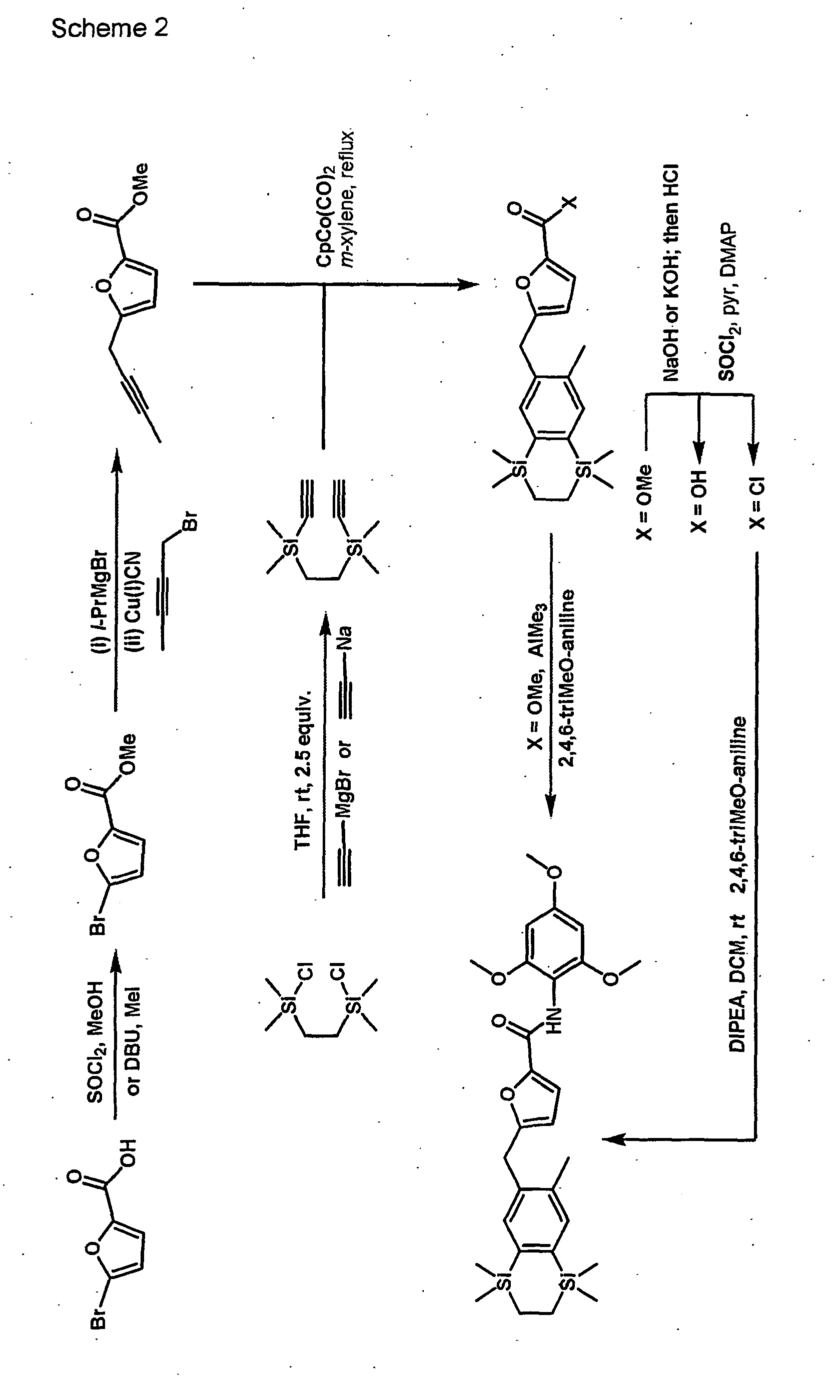

[In the Schemes above, Scheme 1 depicts the synthesis of 2,4,6-trimethoxyaniline, an intermediate used in Scheme 2.]

It will be understood that the processes detailed above are solely for the purpose of illustrating the invention and should not be construed as limiting. A process utilising similar or analogous reagents and/or conditions known to one skilled in the art may also be used to obtain a compound of the invention.

Any mixtures of final products or intermediates obtained can be separated on the basis of the physico-chemical differences of the constituents, in known manner, into the pure final products or intermediates, for example by chromatography, distillation, fractional crystallisation, or by formation of a salt if appropriate or possible under the circumstances.

The activity and selectivity of the compounds may be determined by any suitable assay known in the art; see, for example, Millar *et al,* Methods in Neurosciences : Receptor Molecular Biology Volume 25 (edited by Conn M P et al), 1995, pages 145-162.

The compounds of the invention may be used in the treatment or prevention of numerous ailments, conditions and diseases including, but not limited thereto, cancer, fertility disorders, HIV infection, AIDS, Alzheimer's disease fibrosis, endometriosis, uterine fibroids, uterine leiomyoma and the like.

The term "cancer" as used herein refers to any disease or condition characterised by uncontrolled, abnormal growth of cells and includes all known types of cancer, for example cancer of the bladder, breast, colon, brain, bone, head, blood, eye, neck, skin, lungs, ovaries, prostate and rectum; digestive, gastrointestinal, endometrial, hematological, AIDS-related, muscoskeletal, neurological and gynecological cancers; lympomas, melanomas and leukaemia. The term refers to both solid and liquid tumours.

In therapeutic use, the active compound may be administered orally, intravenously, rectally, parenterally, by inhalation (pulmonary delivery), topically, ocularly, nasally, or to the buccal cavity. Oral administration is preferred. Thus, the therapeutic compositions of the present invention may take the form of any of the known pharmaceutical compositions for such methods of administration. The compositions may be formulated in a manner known to those skilled in the art so as to give a controlled release, for example rapid release or sustained release, of the compounds of the present invention. Pharmaceutically acceptable carriers suitable for use in such compositions are well known in the art. The compositions of the invention may contain 0.1-99% by weight of active compound. The compositions of the invention are generally prepared in unit dosage form. Preferably, a unit dose comprises the active ingredient in an amount of 1-500 mg. The excipients used in the preparation of these compositions are the excipients known in the art.

Appropriate dosage levels may be determined by any suitable method known to one skilled in the art. It will be understood, however, that the specific dose level for any particular patient will depend upon a variety of factors including the activity of the specific compound employed, the age, body weight, general health, sex, diet, time of administration, route of administration, rate of excretion, drug combination and the severity of the disease undergoing treatment.

Compositions for oral administration are preferred compositions of the invention and there are known pharmaceutical forms for such administration, for example tablets, capsules, granules, syrups and aqueous or oily suspensions. The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions, and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example corn starch or alginic acid; binding agents, for example starch gelatin, acacia, microcrystalline cellulose or polyvinyl pyrrolidone; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long-chain aliphatic alcohols, for example heptadecaethyleneoxycetanol, or condensation products of ethylene oxide with partial esters derived from fatty acids, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable sweetening, flavouring and colouring agents may also be present.

The pharmaceutical compositions of the invention may also be in the form of oil-in-water emulsions. The oily phase may be a vegetable oil, for example olive oil or arachis oil, or a mineral oil, for example liquid paraffin, or mixtures of these. Suitable emulsifying agents may be naturally occurring gums, for example gum acacia or gum tragacanth, naturally occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid, find use in the preparation of injectables.

The compounds of the.invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Compositions for topical administration are also suitable for use in the invention. The pharmaceutically active compound may be dispersed in a pharmaceutically acceptable cream, ointment or gel. A suitable cream may be prepared by incorporating the active compound in a topical vehicle such as light liquid paraffin, dispersed in a aqueous medium using surfactants. An ointment may be prepared by mixing the active compound with a topical vehicle such as a mineral oil or wax. A gel may be prepared by mixing the active compound with a topical vehicle comprising a gelling agent. Topically administrable compositions may also comprise a matrix in which the pharmaceutically active compounds of the present invention are dispersed so that the compounds are held in contact with the skin in order to administer the compounds transdermally.

The following Example illustrates the invention.

The Example compound and, in some instances, the intermediate compounds may be obtained by more than one route.

All syntheses, except those of Intermediates 1, 2, 4 and 7 were carried out under dry nitrogen. Tetrahydrofuran, diethyl ether, dichloromethane, toluene, and *m*-xylene were dried and purified according to standard procedures and stored under nitrogen. Silica gel TLC plates (Merck 1.05554) were used for the TLC analyses. 2,4,6-Trimethoxybenzamide was prepared according to F. Effenberger et *al, Chem. Ber.* **1968,** *101*, 502-511 and isolated, after recrystallisation from methanol at 4°C, as the methanol-solvate mp 187-188°C. Anal. Calcd for C₁₀H₁₃NO₄·CH₄O: C, 54.31; H, 7.04; N, 5.76. Found: C, 54.2; H, 6.9; N, 5.8.

All ¹H NMR spectra were run at 400 MHz using CDCl₃. LC-MS spectra were run using Conditions A1, A2 or B:
Conditions A1 and A2; mass spectrometer - electrospray source operating in positive and negative ion mode. System running at 1.5 ml/min, detection mode was through a Hexa-pole Mass Spectrometry detector and a Diode-Array detector for UV.
Mobile phase: acetonitrile-water (running from 5 - 95% acetonitrile) with either 0.05% formic acid (Conditions A1) or 0.05% ammonium hydroxide (Conditions A2) added.
Conditions B: mass spectrometer - electrospray source operating in positive and negative ion mode. System running at 2.0 ml/min, 200 ml split to the ESI source with inline DAD detection and SEDEX ELS detection.
Mobile phase: (A) Water with 0.1 % formic acid added, (B) acetonitrile with 0.1 % formic acid added.

**Gradient:**

| Time | flow | %A | % B |
|---|---|---|---|
| 0.00 | 2.0 | 95 | 5 |
| 0.50 | 2.0 | 95 | 5 |
| 4.50 | 2.0 | 5 | 95 |
| 5.00 | 2.0 | 5 | 95 |
| 5.50 | 2.0 | 95 | 5 |
| Column: Luna 3µ C18(2) 30 x 4.6mm | | | |

### Intermediate 1: 2,4,6-Trimethoxyaniline

### Method A

Bromine (8.16 g, 51.1 mmol) was added dropwise at -5°C within 5 min to a stirred solution of sodium hydroxide (9.86 g, 247 mmol) in water (83 mL), followed by the addition of 2,4,6-trimethoxybenzamide (10.0 g, 41.1 mmol) at 0°C in one single portion. The resulting mixture was allowed to warm to 15°C within 2 hours and was then stirred at 20°C for 3 days (change of colour from colourless to dark-brown). The mixture was cooled to 0°C, sodium sulphite (822 mg, 6.52 mmol) was added, and the mixture was then acidified by the addition of 12 M hydrochloric acid (to pH 2), followed by extraction with ethyl acetate (150 mL). The organic phase was discarded, the pH of the aqueous phase was adjusted to pH 14 by addition of a 50% aqueous potassium hydroxide solution, and the mixture was then saturated with potassium carbonate. The resulting pasty precipitate was separated by suction filtration, the pasty filter cake was washed with ethyl acetate (3 x 50 mL), the organic wash solutions were separated, and the aqueous filtrate was extracted with ethyl acetate (2 x 100 mL). The organic wash solutions and the organic extracts were combined and dried over anhydrous sodium sulphate, and the solvent was removed under reduced pressure (rotary evaporator; 200 mbar/40°C). The dark-brown residue was purified by bulb-to-bulb distillation (Kugelrohr apparatus, 140-180°C/2 mbar) to give 2,4,6-trimethoxyaniline in 18% yield as a colourless liquid (1.35 g, 7.37 mmol) which crystallised at 4°C; mp 29-31 °C. Anal. Calcd for C₉H₁₃NO₃: C, 59.00; H, 7.15; N, 7.65. Found: C, 58.9, H, 7.0; N, 7.8.

### Method B

A 12 M hydrochloric acid solution (84.0 mL, 1.01 mol of HCl) was added dropwise at 20°C within 40 minutes to potassium permanganate (12.8 g, 81.0 mmol) (temperature increase), and the resulting chlorine gas was passed through a stirred solution of potassium hydroxide (50.5 g, 900 mmol) in water (300 mL) at 0°C. After the addition of the hydrochloric acid was complete, the residual chlorine gas was passed into the aqueous solution by a nitrogen gas stream for 45 minutes, followed by the addition of 2,4,6-trimethoxybenzamide (48.7 g, 200 mmol) at 0°C in one single portion. The mixture was stirred at 0°C for a further 6 hours and then at 20°C for 12 hours (change of colour from colourless to dark brown), followed by the addition of sodium sulphite (12.7 g, 101 mmol) at 20°C in one single portion: The mixture was stirred at 20°C for 15 minutes, the resulting precipitate.was separated by suction filtration, and the filter cake was washed successively with water (100 mL) and diethyl ether (200 mL). The filtrate and the wash solutions were combined, the two-phase mixture was shaken thoroughly, the organic layer was separated, and the aqueous phase was extracted with diethyl ether (2 x 200 mL). The combined organic extracts were dried over anhydrous sodium sulphate, the solvent was removed under reduced pressure (rotary evaporator, 800 mbar/40°C), and the dark-brown residue was purified by bulb-to-bulb distillation (Kugelrohr apparatus, 110-140°C/0.1 mbar) to give 2,4,6-trimethoxyaniline in 38% yield as a colourless liquid (13.9 g, 75.9 mmol) which crystallised at 4°C; mp 29-31°C. Anal.Calcd for C₉H₁₃NO₃: C, 59.00; H, 7.15; N, 7.65. Found: C, 58.9; H, 7.1; N, 7.5.

### Intermediate 2: 2,4,6-Trimethoxyaniline Hydrochloride

A 2 M ethereal solution of hydrogen chloride (15.5 mL, 31.0 mmol of HCl) was added dropwise at 20°C to a stirred solution of Intermediate 1(5.34 g, 29.1 mmol) in dichloromethane (110 mL), and the mixture was shaken briefly and then kept undisturbed at 20°C for 1 hour (formation of a precipitate). The mixture was then kept undisturbed at 4°C for 16 hours, and the product was isolated by suction filtration, washed with diethyl ether (20 mL), and dried in vacuo (0.01 mbar, 20°C, 4 hours) to give the title compound in 98% yield as a colourless crystalline solid (6.28 g, 28.6 mmol); mp 241-242°C (dec). Anal. Calcd for C₉H₁₄CINO₃: C, 49.21; H, 6.42; N, 6.38. Found: C, 49.2; H, 6.3; N, 6.3:

### Intermediate 3: 1,2-Bis(ethynyldimethylsilyl)ethane

### Method A

This compound can be synthesised according to T. Kusumoto *et al*, *Chem. Lett.* 1988, 1149-1152.

### Method B

A mixture of 1,2-bis(chlorodimethylsilyl) ethane (121 g, 562 mmol), sodium acetylide (300 g of an 18% suspension of NaC≡CH in xylene (mixture of isomers), 1.12 mol of NaC≡CH), and tetrahydrofuran (360 mL) was heated under reflux for 3 hours. The mixture was allowed to cool to 20°C and was then washed with water (2 x 450 mL), and the organic layer was separated. The aqueous wash solutions were extracted with diethyl ether (2 x 300 mL) in the same order as they had been used for workup, and all organic extracts were combined and dried over anhydrous sodium sulphate. Most of the solvent was removed under reduced pressure (rotary evaporator; 800 to 100 mbar, 40°C), and the remaining xylene was then removed by vacuum distillation (30-50°C/15 mbar) using a Vigreux column (40 cm). The residues from four identical runs of this preparation were combined and distilled in vacuo (Vigreux column, 40 cm) to give 245 g of pure 1,2-bis(ethynyldimethylsilyl)ethane (75-77°C/20 mbar) and 126 g of a lower-boiling fraction containing the product and xylene (50-75°C/20 mbar). The latter fraction was redistilled (Vigreux column, 80 cm) to give a further 65 g of the title compound (75-77°C/20 mbar). 1,2-Bis(ethynyldimethylsilyl)ethane was obtained in a total yield of 71 % as a colourless liquid (310 g, 1.59 mol). Anal. Calcd for C₁₀H₁₈Si₂: C, 61.78; H, 9.33. Found: C, 61.6; H, 9.2.

### Intermediate 4: Methyl 5-Bromo-2-furoate

### Method A

A mixture of dichloromethane (700 mL), 5-bromo-2-furoic acid (146 g, 764 mmol), 1,8-diazabicyclo[5.4.0]undec-7-ene (DBU; 122 g, 801 mmol), and iodomethane (130 g, 916 mmol) was heated under reflux for 22 hours. The mixture was allowed to cool to 20°C and was then successively washed with a saturated aqueous ammonium chloride solution (300 mL), a saturated aqueous sodium hydrogen carbonate solution (300 mL), and water (300 mL). The organic layer was separated and the aqueous wash solutions were extracted with ethyl acetate (2 x 200 mL) in the same order as they had been used for workup. All organic extracts were combined and dried over anhydrous sodium sulphate, the solvent was removed under reduced pressure (rotary evaporator; 800 to 150 mbar, 40°C), and the residue was dried in vacuo (10 mbar, 20°C, 30 minutes). n-Hexane (1.1 L) was added to the residue and the resulting mixture was heated under reflux for 5 minutes, followed by filtration of the hot mixture. The filter cake was washed with boiling *n*-hexane (160 mL), the filtrate and wash solution were combined, and the resulting solution was allowed to cool to 20°C within 2 hours and was then kept undisturbed at 4°C for 2 days (formation of a crystalline precipitate). The solid was separated by decantation and recrystallised from boiling n-hexane (720 mL; crystallisation at 4°C over a period of 4 days). The product was again isolated by decantation and dried in vacuo (0.01 mbar, 20°C, 4 hours) to give 112 g of a yellowish crystalline solid. The mother liquours of the crystallisation steps were combined and concentrated to 250 mL (rotary evaporator, 300 mbar/40°C), and a further 17 g of the product were obtained by crystallisation using the same method as described above. The title compound was obtained in a total yield of 82% as a yellowish crystalline solid (129 g, 629 mmol); mp 64-65°C. Anal. Calcd for C₆H₅BrO₃: C, 35.15; H, 2.46; Br, 38.98. Found: C, 35.4; H, 2.7; Br, 38.8.

### Method B

To a solution of 5-bromo-2-furoic acid (20 g, 0.105 mol) in methyl alcohol (150 mL) was added thionyl chloride (5 mL) and the reaction was stirred under an inert atmosphere. After 20 hours the mixture was evaporated under reduced pressure, co-evaporated with methyl alcohol (2 x 50 mL) and taken up into ethyl acetate (200 mL). This was washed with saturated aqueous sodium bicarbonate solution (50 mL), which was then re-extracted into ethyl acetate (2 x 20 mL). The combined organic phases were dried (sodium sulfate) and evaporated under reduced pressure to afford the title compound as an off-white amorphous solid that was used as received without further purification (25.8 g). ¹H NMR δ 7.11 (1 H, d, J = 3.5 Hz), 6.44 (1 H, d, J = 3.5 Hz) and 3.89 (3 H, s). LC-MS (Conditions A1); Rₜ = 3.43 minutes.

### Intermediate 5: Methyl 5-(but-2-ynyl)-2-furoate

### Method A

A 0.68 M solution of isopropylmagnesium bromide in tetrahydrofuran (552 mL, 375 mmol of *i*-PrMgBr) was added dropwise within 110 minutes at -40°C (±5°C; temperature measured within the flask) to a solution of **Intermediate 4** (70.0 g, 341 mmol) in tetrahydrofuran (1.0 L). The resulting mixture was stirred at -40°C (±5°C) for a further 3 hours, followed by sequential addition of copper(I) cyanide (7.70 g, 86.0 mmol) in one single portion and of 1-bromo-2-butyne (64.8 g, 487 mmol) within 5 minutes (temperature increase to -20°C). The mixture was stirred for 2 hours at -35°C and kept undisturbed at -20°C for a further 16 hours, and the cold (-20°C) mixture was then added to a cold (0°C) vigorously stirred emulsion consisting of a saturated aqueous ammonium chloride solution (400 mL) and ethyl acetate (200 mL). The resulting heterogenous mixture was stirred for 30 minutes at 0°C, followed by filtration at the same temperature. The filter cake was washed with ethyl acetate (2× 100 mL), and the two-phase filtrate and the wash solutions were combined. The organic layer was separated, the aqueous phase was extracted with ethyl acetate (3 x 100 mL), and the organic extracts were combined and then dried over anhydrous sodium sulphate. The solvent was removed under reduced pressure (rotary evaporator; 300 to 150 mbar, 40°C), and the residue was purified by bulb-to-bulb distillation (Kugelrohr apparatus; first fraction (<100°C, 1.03 g), discarded; second fraction (100-130°C, 51.7 g), crude product). The second fraction (yellowish oil) was crystallised from boiling n-hexane (265 mL, crystallisation at 4°C over a period of 4 days), and the crystalline solid was separated by decantation and recrystallised from boiling n-hexane (190 mL; crystallisation at 4°C for 2 days). The product was again isolated by decantation and dried in vacuo (0.01 mbar, 20°C, 4 hours) to give 34.2 g of a colourless crystalline solid. The mother liquours of the crystallisation steps were combined, the solvent was removed under reduced pressure (rotary evaporator, 300 mbar/40°C), and a further 3.4 g of the product were obtained by crystallisation of the oily residue using the same method as described above. The title compound was obtained in a total yield of 62% as a colourless crystalline solid (37.6 g, 211 mmol); mp 44°C. Anal. Calcd for C₁₀H₁₀O₃: C, 67.41; H, 5.66. Found: C, 67.3; H, 5.7.

### Method B

A solution of Intermediate 4 (1.95 g, 9.56 mmol) in dry tetrahydrofuran (THF, 50 ml) was stirred under nitrogen and cooled to -35 °C. To this was added a solution of *iso*-propyl magnesium bromide (15.27 ml, 0.66 M) dropwise over a period of 10 minutes. The reaction was then stirred for 1 hour at -35 °C before copper (I) cyanide (215 mg; 2.42 mmol) and 1-bromo-2-butyne (1.17 ml, 13.38 mmol) were added consecutively and stirring was continued for 1.5 hours. The resulting mixture was then treated with saturated aqueous ammonium chloride solution (25 ml) maintaining the temperature below -30 °C before the mixture was allowed to slowly warm to room temperature. The resulting precipitate was removed by vacuum filtration and the solid washed with ethyl acetate (3 x 20 mL). The aqueous phase was separated and extracted into ethyl acetate (3 x 20 mL) before the combined organic phases were dried (sodium sulfate) and evaporated under reduced pressure. The tan-coloured gum was purified by chromatography (silica, ethyl acetate-petroleum ether; 1:9) to afford the title compound as a pale yellow oil (944 mg, 55 % yield). ¹H NMR δ 7.13 (1 H, d, J = 3.5 Hz), 6.38 (1 H, d, *J* = 3.5 Hz), 3.89 (3 H, s), 3.62 (2 H, m) and 1.82 (3 H, m). LC-MS (Conditions B); Rₜ =3.65 minutes; m/z [M+H]⁺ 179.

### Intermediate 6: Methyl 5-[(3,5,5,8,8-pentamethyl-5,8-disila-5,6,7,8-tetrahydro-2-naphthyl)methyl]-2-furoate

### Method A

A solution of cyclopentadienylcobaltdicarbonyl (CpCo(CO)₂; 1.53 g, 8.50 mmol) in *m*-xylene (90 mL) was added dropwise within 14 hours to a boiling solution of **Intermediate 3** (10.9 g, 56.1 mmol) and **Intermediate 5** (10.0 g, 56.1 mmol) in *m*-xylene (100 mL), followed by addition of **Intermediate 3** (5.47 g, 28.1 mmol) in one single portion at 20°C and then by dropwise addition of a solution of CpCo(CO)₂ (1.03 g, 5.72 mmol) in *m*-xylene (60 mL) within 12 hours at reflux temperature. (To avoid heating of the CpCo(CO)₂ solution before its addition, the dropping funnel containing this catalyst was separated from the refluxing reaction mixture by a glass tube (length, 20 cm), through which the CpCo(CO)₂ solution was allowed to drop freely into the refluxing mixture.) The solvent was removed under reduced pressure (rotary evaporator, 20 mbar/40°C), and the residue was purified by column chromatography (column diameter, 4.5 cm; column length, 65 cm; silica gel (15-40*µ*m, Merck 1.15111), 425 g; eluent, ethyl acetate/*n*-hexane 5:95 (v/v)). The relevant fractions were combined, the solvent was removed under reduced pressure (rotary evaporator; 350 to 150 mbar, 40°C), and the residue was dried in vacuo (0.01 mbar, 20°C, 1 hour) to give 11.1 g of a yellowish oil which was redissolved in n-hexane (95 mL). The product was crystallised at -20°C over a period of 7 days and then isolated by decantation, washed with cold (-20°C) *n*-hexane (70 mL), and dried in vacuo (0.001 mbar, 20°C, 2 hours) to give 6.83 g of a colourless crystalline solid. The mother liquour was concentrated under reduced pressure (rotary evaporator, 300 mbar/40°C) to a volume of 25 mL and a further 1.28 g of the product were obtained by crystallisation using the same method as described above. The title compound was obtained in a total yield of 39% as a colourless crystalline solid (8.11 g, 21.8 mmol); mp 75-76°C. Anal. Calcd for C₂₀H₂₈O₃Si₂: C, 64.47; H, 7.57. Found: C, 64.2; H, 7.7.

### Method B

A mixture of **Intermediate 5** (216 mg, 1.2 mmol) and **Intermediate 3** (236 mg, 1.2 mmol) in dry *m*-xylene (25 mL) was purged with nitrogen (15 minutes) before being heated to reflux under an inert atmosphere. To this boiling mixture was added cyclopentadienylcobaltdicarbonyl (11 mg, 0.5 mol%) in dry *m*-xylene (25 mL, solvent also purged with nitrogen before use) via a syringe pump over 9 hours. The reaction was heated overnight before a second solution of the catalyst (0.5 mol%) in *m*-xylene (25 ml) was added drop-wise over a period of 4 hours. The reaction mixture was then allowed to cool, evaporated under reduced pressure and purified by column chromatography (silica; ethyl acetate-petroleum ether; 1:19) to afford the desired material (112 mg, 25 % yield). ¹H NMR δ 7.30 (2 H, s), 7.12 (1 H, d, J = 3.3 Hz), 5.96 (1 H, d, J = 3.3 Hz), 4.40 (2 H, s), 3.91 (3 H, s), 2.29 (3 H, s), 0.99 (4 H, s), 0.26 (6 H, s) and 0.22 (6 H, s). LC-MS (Conditions B); Rₜ = 5.20 minutes.

### Intermediate 7: 5-[(3,5,5,8,8-Pentamethyl-5,8-disila-5,6,7,8-tetrahyrdro-2-naphthyl)methyl]-2-furoic Acid

### Method A

A mixture of water (48 mL), methanol (143 mL), potassium hydroxide (6.90 g, 123 mmol), and Intermediate 6.(4.34 g, 11.6 mmol) was heated under reflux for 20 minutes (slow dissolution). The mixture was then stirred at 0°C for 1 minute, followed by addition of dichloromethane (50 mL) and 1 M hydrochloric acid to adjust to a pH of 1. The resulting two-phase mixture was stirred at 0°C for 5 minutes and then at 20°C for a further 15 minutes. The organic phase was separated, the aqueous layer was extracted with dichloromethane (3 x 50 mL), and the organic extracts were combined and dried over anhydrous sodium sulphate. The solvent was removed under reduced pressure (rotary evaporator, 800 mbar/40°C), and the solid residue was dried in vacuo (0.01 mbar, 20°C, 1 hour) and then dissolved in diethyl ether (140 mL). The product was crystallised by vapour diffusion of *n*-pentane into this solution at 20°C over a period of 14 days and was then isolated by decantation, washed with *n*-pentane (20mL), and dried in vacuo (0.01 mbar, 20°C, 4 hours) to give 3.42 g of a colourless crystalline solid. The solvent of the mother liquour was removed under reduced pressure (rotary evaporator, 800 mbar/40°C), the residue was redissolved in diethyl ether (30 mL), and a further 420 mg of the' product were obtained by crystallisation using the same method as described above. The title compound was obtained in a total yield of 92% as a colourless crystalline solid (3.84 g, 10.7 mmol); mp 171°C. Anal. Calcd for C₁₉H₂₆O₃Si₂: C, 63.64; H, 7.31. Found: C, 63.8; H, 7.3.

### Method B

To a solution of **Intermediate 6** (200 mg, 0.56 mmol) in ethyl alcohol (20 mL) was added aqueous sodium hydroxide solution (1 M, 0.614 mL) and the mixture was heated to reflux for 2 hours before being concentrated *in vacuo.* Water (25 mL) was added before the resulting mixture was acidified with dilute hydrochloric acid (1 M, to pH 2) and extracted with ethyl acetate (3 x 50 mL). The combined organic extracts were dried (sodium sulfate) and evaporated under reduced pressure to afford a tan solid which was purified by flash column chromatography (silica, ethyl acetate-light petroleum; 1:9 containing 1 % acetic acid) to afford the desired material (112 mg, 56 % yield). ¹H NMR δ 7.32 (2 H, m), 7.10 (1 H, d, J = 3.5 Hz), 5.98 (1 H, d, J = 3.5 Hz); 3.89 (2 H, s), 2.29 (3 H, s); 1.01 (4 H, s), 0.26 (6 H, s) and 0.22 (6 H, s). LC-MS (Conditions A1) Rₜ = 4.80 minutes, m/z 359 [M+H]⁺.

### Example: 5-[(3,5,5,8,8-Pentamethyl-5,8-disila-5,6,7,8-tetrahydro-2-naphthyl)methyl]-N-(2,4,6-trimethoxyphenyl)furan-2-carboxamide

### Method A

A 2.0 M solution of trimethylaluminium in toluene (5.00 mL, 10.0 mmol of AIMe₃) was added dropwise at -30°C within 8 minutes to a stirred suspension of **Intermediate 2** (2.20 g, 10.0 mmol) in toluene (20 mL) (dissolution of **Intermediate 2,** followed by the formation of a precipitate). The stirred mixture was allowed to warm to -20°C within 25 minutes and then to 20°C within a further 1 hour (dissolution of the precipitate), and the resulting solution was then added dropwise at 0°C within 10 minutes to a stirred solution of **Intermediate 6** (1.86 g, 4.99 mmol) in dichloromethane (20 mL). The resulting mixture was stirred at 0°C for a further 1 hour and then at 20°C for 3 days (quantitative conversion (HPLC control), change of colour from colourless to black), followed by the addition of a half-saturated aqueous ammonium acetate solution (100 mL) (formation of a precipitate). The precipitate was removed by filtration and washed with ethyl acetate (5 x 20 mL), the filtrate and wash solutions were combined, and the organic layer was separated and washed with water (100 mL). The resulting aqueous layers were extracted with ethyl acetate (3 x 50 mL) in the same sequence as they had been used for workup. All organic extracts were combined and dried over anhydrous sodium sulphate, the solvent was removed under reduced pressure (rotary evaporator; 800 to 80 mbar, 40°C), and the residue (4.0 g of a dark brown viscous oil) was purified by column chromatography (column diameter, 3.0 cm; column length, 80 cm; silica gel (32-63 µm, **ICN** 02826), 230 g; eluent, ethyl acetate/*n*-hexane 55:45 (v/v)). The relevant fractions (TLC, ethyl acetate/*n*-hexane 55:45 (v/v), *R*_{f} = 0.42) were combined, the solvent was removed under reduced pressure (rotary evaporator; 350 to 150 mbar, 40°C), and the residue was dried in vacuo (0.001 mbar, 20°C, 1 hour) to give 2.77 g of a brown oil. The product was crystallised and then recrystallised from diethyl ether (54 mL for each crystallisation (sonication was necessary to achieve complete dissolution); crystallisation at 4°C over a period of 1 day and then at -20°C over a period of 3 days). The crystalline solid was isolated by decantation, washed with n-pentane (5 mL), and dried in vacuo (0.001 mbar, 20°C, 4 hours) to give 1.58 g of the title compound. The solvent of the combined mother liquours was removed to yield 1.06 g of a brown oily product which was crystallised twice from diethyl ether (19 mL) to give a further 580 mg of the desired material. 5-[(3,5,5,8,8-pentamethyl-5,8-disila-5,6,7,8-tetrahydro-2-naphthyl)methyl]-*N*-(2,4,6-trimethoxyphenyl)furan-2-carboxamide was obtained in a total yield of 83% as a colourless crystalline solid (2.16 g, 4.12 mmol); mp 139°C. Anal. Calcd for C₂₈H₃₇NO₅Si₂: C, 64.21; H, 7.12; N, 2.67. Found: C, 64.2; H, 7.1; N, 2.7.

### Method B

*Solution a.* A solution of **Intermediate 7** (1.00 g, 2.79 mmol) and thionyl chloride (6.85 g, 57.6 mmol) in dichloromethane (9 mL) was heated under reflux for 5 hours. All volatile components were removed in vacuo (0.001 mbar, 20°C, **1** hour), and the oily residue was redissolved in dichloromethane (4 mL). *Solution b.* **Intermediate 1** (572 mg, 3.12 mmol; freshly distilled by bulb-to-bulb distillation directly before use), pyridine (245 mg, 3.10 mmol), and 4-(dimethylamino)pyridine (DMAP; 7.0 mg, 57 µmol) were dissolved in dichloromethane (5 mL).

*Solution* a was added dropwise at 0°C within 10 minutes to the stirred *solution b* (formation of a precipitate which redissolved later; change of colour from colourless to orange). The mixture was stirred at 0°C for a further 10 minutes and then at 20°C for 16 hours, followed by addition of dichloromethane (20 mL). The resulting solution was washed with a 5 vol-% aqueous acetic acid solution (20 mL), a half-saturated aqueous sodium hydrogen carbonate solution (20 mL), and water (20 mL). The organic layer was separated, and the aqueous wash solutions were extracted with dichloromethane (2 x 20 mL) in the same order as they had been used for workup. The organic extracts were combined and dried over anhydrous sodium sulphate, the solvent was removed under reduced pressure (rotary evaporator, 800 mbar/40°C), and the residue was dried in vacuo (10 mbar, 20°C, 30 minutes) and then purified by column chromatography (column diameter, 3.5 cm; column length, 66 cm; silica gel (15-40 µm, Merck 1.15111), 235 g; eluent, ethyl acetate/*n*-hexane 55:45 (v/v)). The relevant fractions (TLC, ethyl acetate/*n*-hexane 55:45 (v/v), *R*_{f} = 0.42) were combined, the solvent was removed under reduced pressure (rotary evaporator; 350 to 150 mbar, 40°C), and the residue was dried in vacuo (0.01 mbar, 20°C, 1 hour) to give 548 mg of an oil which was dissolved in diethyl ether (5 mL). The product crystallised from the resulting solution at 4°C within 7 days, and the crystalline solid was isolated by decantation, washed with n-pentane (2 mL), and dried in vacuo (0.01 mbar, 20°C, 4 hours) to give 5-[(3,5,5,8,8-pentamethyl-5,8-disila-5,6,7,8-tetrahydro-2-naphthyl)methyl]-N-(2,4,6-trime'thoxyphenyl)furan-2-carboxamide in 22% yield as a colourless crystalline solid (318 mg, 607 µmol); mp 139-140°C. Anal. Calcd for C₂₈H₃₇NO₅Si₂: C, 64.21; H, 7.12; N, 2.67. Found: C, 64.6; H, 7.1; N, 2.7.

### Method C

A solution of **Intermediate 7** (584 mg, 1.63 mmol) in dichloromethane (10 mL) was added dropwise at 20°C within 10 minutes to a stirred solution of dicyclohexylcarbodiimide (DCC; 370 mg, 1.79 mmol) and pyridine (265 mg, 3.35 mmol) in dichloromethane (10 mL). The mixture was stirred at 20°C for a further 24 hours, followed by addition of DMAP (4.0 mg, 33 µmol) in one single portion. Subsequently, a solution of **Intermediate 1** (358 mg, 1.95 mmol; freshly distilled by bulb-to-bulb distillation directly before use) in dichloromethane (5 mL) was added to the stirred mixture at 20°C within 10 minutes, and stirring was continued for a further 3 days at 20°C (formation of a precipitate). The mixture was then washed with water (2 x 25 mL), the organic layer was separated, and the aqueous wash solutions were extracted with dichloromethane (2 x 20 mL) in the same order as they had been used for workup. The organic extracts were combined and dried over anhydrous sodium sulphate (remaining solids that did not dissolve in neither phase were filtered off along with the sodium sulphate), the solvent was removed under reduced pressure (rotary evaporator, 800 mbar/40°C), and the residue was dried in vacuo (10 mbar, 20°C, 30 minutes) and then purified by column chromatography (column diameter, 3.5 cm; column length, 70 cm; silica gel (15-40 µm, Merck 1.15111), 250 g; eluent, ethyl acetate/*n*-hexane 55:45 (v/v)). The relevant fractions (TLC, ethyl acetate/*n-*hexane 55:45 (v/v), *R*_{f} = 0.42) were combined, the solvent was removed under reduced pressure (rotary evaporator; 350 to 150 mbar, 40°C), and the residue was dried in vacuo (0.01 mbar, 20°C, 1 hour) to give 454 mg of an oil which was dissolved in diethyl ether (8 mL). The resulting solution was then kept undisturbed at 4°C for 2 days and at -20°C for a further 4 days (formation of a precipitate). The crystalline solid was isolated by decantation, washed with n-pentane (3 mL), and dried in vacuo (0.01 mbar, 20°C, 4 hours) to give 5-[(3,5,5,8,8-pentamethyl-5,8-disila-5,6,7,8-tetrahydro-2-naphthyl)methyl]-*N*-(2,4,6-trimethoxyphenyl)furan-2-carboxamide in 37% yield as a colourless crystalline solid (318 mg, 607 µmol); mp 139 °C. Anal. Calcd for C₂₈H₃₇NO₅Si₂: C, 64.21; H, 7.12; N, 2.67. Found: C; 64.2; H, 7.1; N, 2.7.

### Method D

To a stirred solution of **Intermediate 7** (215 mg, 0.624 mmol) in dry dichloromethane (10 mL) under an inert atmosphere was added thionyl chloride (0.75 mL, 10.2 mmol) and dry *N,N*-dimethylformamide (3 drops). The unstirred solution was left in the dark for 3 days. The reaction mixture was then evaporated under reduced pressure and co-evaporated with toluene (2 x 10 mL). To a solution of this acid chloride in dry dichloromethane (10 mL) was added triethylamine (0.416 ml, 1.03 mmol) and **Intermediate 1** (216 mg, 1.19 mmol). After stirring for 48 hours, the reaction mixture was diluted with dichloromethane (20 mL) and washed with saturated aqueous sodium bicarbonate solution (25 mL), which was then re-extracted with dichloromethane (2 x 10 mL). The combined organic extracts were then dried (magnesium sulphate) and evaporated under reduced pressure to give a light tan coloured solid which was purified by column chromatography (alumina, 10% methyl alcohol-dichloromethane) to afford the desired product (76 mg, 23% yield) as a light pink foam m.p. 134-136 °C (from cyclohexane). ¹H NMR δ 7.31 (1 H, s), 7.27 (1 H, s), 7.08 (1 H, d, *J=* 3.3 Hz), 6.19 (2.H, s), 6.03 (1 H, d, J = 3.3 Hz), 4.02 (2 H, s), 3.83 (3 H, s), 3.82 (6 H, s), 2.33 (3 H, s), 1.0 (4 H, s), 0.23 (6 H, s) and 0.19 (6 H, s). LC-MS (Conditions A1) Rₜ= 4.97 minutes, m/z 524 [M+H]⁺, (Conditions A2) Rₜ = 5.00 minutes, m/z 524 [M+H]⁺.

## Claims

1. A compound of formula (I) 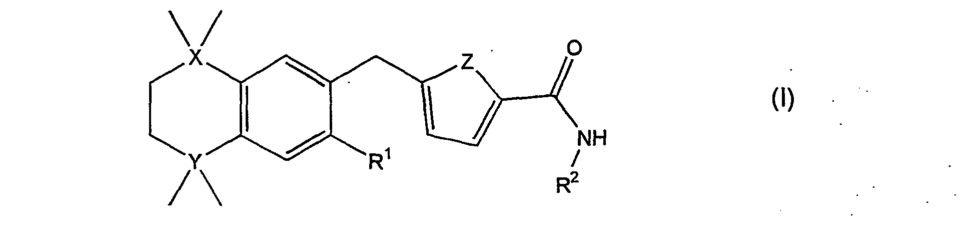 wherein
one of X and Y is silicon, and the other is carbon or silicon;
Z is oxygen, sulphur or -N(R)-, wherein R is hydrogen or alkyl;
R¹ is hydrogen, halogen, alkyl, alkenyl, alkynyl, alkoxy or cycloalkyl; and
R² is alkyl, alkenyl, alkynyl, cycloalkyl, heterocycloalkyl, aryl, heteroaryl, -alkyl-cycloalkyl, -alkyl-heterocycloalkyl, -alkyl-aryl or -alkyl-heteroaryl;
or a pharmaceutically acceptable salt thereof.

2. A compound according to claim 1, wherein X and Y are each silicon.

3. A compound according to claim 1 or claim 2, wherein Z is oxygen.

4. A compound according to any preceding claim, wherein R¹ is hydrogen or alkyl.

5. A compound according to claim 4, wherein R¹ is methyl.

6. A compound according to any preceding claim, wherein R² is aryl, -CH₂-cycloalkyl, -CH₂-aryl, -CH₂-heterocycloalkyl or -CH₂-heteroaryl.

7. A compound according to claim 6, wherein R² is optionally substituted phenyl.

8. A compound according to claim 7, wherein R² is phenyl substituted with one, two or three alkoxy groups.

9. A compound according to claim 1, which is 5-[(3,5,5,8,8-pentamethyl-5,8-disila-5,6,7,8-tetrahydro-2-naphthyl)methyl]-*N*-(2,4,6-trimethoxyphenyl)furan-2-carboxamide.

10. A compound according to any preceding claim, for therapeutic use.

11. A pharmaceutical composition comprising a compound of any of claims 1 to 9 and a pharmaceutically acceptable diluent or carrier.

12. Use of a compound of any of claims 1 to 9, for the manufacture of a medicament for the treatment or prevention of a disease or condition associated with GnRH.

13. Use according to claim 12, for the treatment or prevention of progression of cancer.

14. Use according to claim 13, for leukaemia therapy.

15. Use according to claim 12, for the treatment or prevention of a fertility disorder.

16. Use according to claim 12, for the treatment or prevention of HIV infection or AIDS.

17. Use according to claim 12, for the treatment or prevention of Alzheimer's disease.

18. Use according to claim 12, for the treatment or prevention of fibrosis.

19. Use according to claim 12, for the treatment or prevention of endometriosis.

20. Use according to claim 12, for the treatment or prevention of uterine fibroids.

21. Use according to claim 12, for the treatment or prevention of uterine leiomyoma.

## Patentansprüche

1. Verbindung der Formel (I) 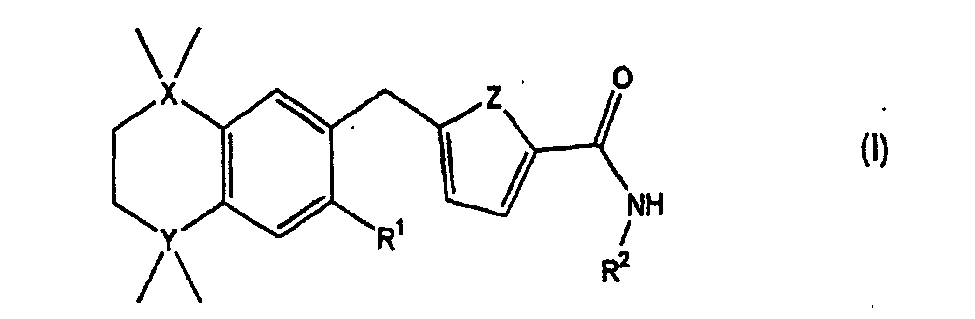 wobei
eines von X und Y Silizium und das andere Kohlenstoff oder Silizium ist;
Z Sauerstoff, Schwefel oder -N(R)- ist, wobei R Wasserstoff oder Alkyl ist;
R¹ Wasserstoff, Halogen, Alkyl, Alkenyl, Alkinyl, Alkoxy oder Cycloalkyl ist; und
R² Alkyl, Alkenyl, Alkinyl, Cycloalkyl, Heterocycloalkyl, Aryl, Heteroaryl, -Alkyl-cycloalkyl, -Alkyl-heterocycloalkyl, -Alkyl-aryl oder Alkyl-heteroaryl ist; oder eine pharmazeutisch verträgliches Salz davon.

2. Verbindung gemäß Anspruch 1, wobei X und Y jeweils Silizium sind.

3. Verbindung gemäß Anspruch 1 oder Anspruch 2, wobei X Sauerstoff ist.

4. Verbindung gemäß einem der vorhergehenden Ansprüche, wobei R¹ Wasserstoff oder Alkyl ist.

5. Verbindung gemäß Anspruch 4, wobei R¹ Methyl ist.

6. Verbindung gemäß einem der vorhergehenden Ansprüche, wobei R² Aryl, -CH₂₋cycloalkyl, -CH₂-aryl, -CH₂-heterocycloalkyl oder -CH₂-heteroaryl ist.

7. Verbindung gemäß Anspruch 6, wobei R² gegebenenfalls mit Phenyl substituiert ist.

8. Verbindung gemäß Anspruch 7, wobei R² Phenyl, substituiert mit einer, zwei oder drei Alkoxygruppen ist.

9. Verbindung gemäß Anspruch 1, die 5-[(3,5,5,8,8-Pentamethyl-5,8-disila-5,6,7,8-tetrahydro-2-naphthyl)methyl]-N-(2,4,6-trimethoxyphenyl)furan-2-carboxamid ist.

10. Verbindung gemäß einem der vorhergehenden Ansprüche, zur therapeutischen Verwendung.

11. Pharmazeutische Zusammensetzung umfassend eine Verbindung nach einem der Ansprüche 1 bis 9 und ein/en pharmazeutisch verträgliches/en Verdünnungsmittel oder Träger.

12. Verwendung einer Verbindung gemäß einem der Ansprüche 1 bis 9, für die Herstellung eines Medikaments für die Behandlung oder Prävention einer Erkrankung oder eines Zustandes, die/der mit GnRH assoziiert ist.

13. Verwendung gemäß Anspruch 12, für die Behandlung oder Prävention der Progression von Krebs.

14. Verwendung gemäß Anspruch 13, für die Leukämietherapie.

15. Verwendung gemäß Anspruch 12, für die Behandlung oder Prävention einer Fruchtbarkeitsstörung.

16. Verwendung gemäß Anspruch 12, für die Behandlung oder Verhinderung einer HIV Infektion oder AIDS.

17. Verwendung gemäß Anspruch 12, für die Behandlung oder Prävention von Alzheimer'scher Erkrankung.

18. Verwendung gemäß Anspruch 12, für die Behandlung oder Prävention von Fibrose.

19. Verwendung gemäß Anspruch 12, für die Behandlung oder Prävention von Endometriose.

20. Verwendung gemäß Anspruch 12, für die Behandlung oder Prävention von Gebährmutterfibromen.

21. Verwendung gemäß Anspruch 12, für die Behandlung oder Prävention von Gebärmutterleiomyom.

## Revendications

1. Composé de formule (I) 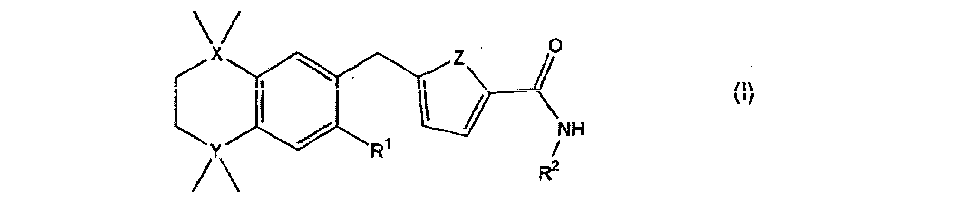 dans laquelle
l'un de X et Y représente le silicium et l'autre représente le carbone ou le silicium ;
Z représente l'oxygène, le soufre ou un groupe -N(R)-, dans lequel R représente l'hydrogène ou un groupe alkyle ;
R¹ représente l'hydrogène, un halogène, un groupe alkyle, alcényle, alcynyle, alkoxy ou cycloalkyle ; et
R² représente un groupe alkyle, alcényle, alcynyle, cycloalkyle, hétérocycloalkyle, aryle, hétéroaryle, -alkyl-cycloalkyle, -alkyl-hétérocycloalkyle, -alkyl-aryle ou -alkyl-hétéroaryle ;
ou un de ses sels pharmaceutiquement acceptables.

2. Composé suivant la revendication 1, dans lequel X et Y représentent chacun le silicium.

3. Composé suivant la revendication 1 ou la revendication 2, dans lequel Z représente l'oxygène.

4. Composé suivant l'une quelconque des revendications précédentes, dans lequel R¹ représente l'hydrogène ou un groupe alkyle.

5. Composé suivant la revendication 4, dans lequel R¹ représente un groupe méthyle.

6. Composé suivant l'une quelconque des revendications précédentes, dans lequel R² représente un groupe aryle, -CH₂₋cycloalkyle, -CH₂-aryle, -CH₂-hétérocycloalkyle ou -CH₂₋hétéroaryle.

7. Composé suivant la revendication 6, dans lequel R² représente un groupe phényle facultativement substitué.

8. Composé suivant la revendication 7, dans lequel R² représente un groupe phényle substitué avec un, deux ou trois groupes alkoxy.

9. Composé suivant la revendication 1, qui est le 5- [(3,5,5,8,8-pentaméthyl-5,8-disila-5,6,7,8-trétrahydro-2-naphtyl)méthyl]-*N*-(2,4,6-triméthoxyphényl)furanne-2-carboxamide.

10. Composé suivant l'une quelconque des revendications précédentes, à usage thérapeutique.

11. Composition pharmaceutique comprenant un composé de l'une quelconque des revendications 1 à 9 et un diluant ou support pharmaceutiquement acceptable.

12. Utilisation d'un composé suivant l'une quelconque des revendications 1 à 9 pour la production d'un médicament destiné au traitement ou à la prévention d'une maladie ou affection associée à GnRH.

13. Utilisation suivant la revendication 12, pour le traitement ou la prévention de la progression du cancer.

14. Utilisation suivant la revendication 13, pour la thérapie de la leucémie.

15. Utilisation suivant la revendication 12, pour le traitement ou la prévention d'un trouble de la fécondité.

16. Utilisation suivant la revendication 12, pour le traitement ou la prévention de l'affection par le VIH ou du SIDA.

17. Utilisation suivant la revendication 12, pour le traitement ou la prévention de la maladie d'Alzheimer.

18. Utilisation suivant la revendication 12, pour le traitement ou la prévention de la fibrose.

19. Utilisation suivant la revendication 12, pour le traitement ou la prévention de l'endométriose.

20. Utilisation suivant la revendication 12, pour le traitement ou la prévention de fibromes utérins.

21. Utilisation suivant la revendication 12, pour le traitement ou la prévention du léiomyome utérin.
